# EUROPEAN PATENT SPECIFICATION

(11) **EP 3 651 647 B1**
(45) Date of publication and mention of the grant of the patent: **07.10.2026**
(21) Application number: 18755331.8
(22) Date of filing: 13.07.2018
(51) Int. Cl.: A61B 5/145, A61M 5/172, A61M 5/142, G16H 20/13, G16H 40/63

(54) **MULTI-SCALE DISPLAY OF BLOOD GLUCOSE INFORMATION**
MULTISKALIGE ANZEIGE VON BLUTGLUKOSEINFORMATIONEN
AFFICHAGE À ÉCHELLES MULTIPLES D'INFORMATIONS DE GLYCÉMIE

(30) Priority: 13.07.2017 US 201762532168 P
(43) Date of publication of application: 20.05.2020
(73) Proprietor: Bigfoot Biomedical, Inc., Milpitas, CA 93035 (US)
(72) Inventor: Desborough, Lane, Thousand Oaks, California 91360 (US); Mazlish, Bryan, Palo Alto, California 94301 (US); Kabel-Eckes, Sabine, Mountain View, California 94043 (US); Boissier, Jeff, San Jose, California 95120 (US)
(74) Representative: Peterreins Schley
(86) International application number: PCT/US2018/042089
(87) International publication number: WO 2019/014594

(56) References cited:
- EP-A1- 2 500 049
- WO-A1-2018/111928
- US-A1- 2009 113 295
- US-A1- 2011 201 911
- US-A1- 2015 227 710
- RODBARD DAVID: "A Semilogarithmic Scale for Glucose Provides a Balanced View of Hyperglycemia and Hypoglycemia", JOURNAL OF DIABETES SCIENCE AND TECHNOLOGY, vol. 3, no. 6, 1 November 2009 (2009-11-01), US, pages 1395 - 1401, XP093174440, ISSN: 1932-2968, Retrieved from the Internet <URL:https://www.ncbi.nlm.nih.gov/pmc/articles/PMC2787040/pdf/dst-03-1395.pdf> DOI: 10.1177/193229680900300620

## Description

### TECHNICAL FIELD

This document relates to a multi-scale display of blood glucose information and, more particularly, to the use of such a display in providing information regarding blood glucose levels of a person with diabetes (PWD).

### BACKGROUND

People with Type I, Type II, or gestational diabetes must track their blood glucose levels and sometimes treat their condition to maintain appropriate blood glucose levels. Control of diabetes can include the monitoring of blood glucose levels using a blood glucose monitor (BGM) and sometimes a continuous glucose monitor (CGM). People with Type I, and some people with Type II or gestational diabetes, require insulin or an analog thereof. Because it cannot be taken orally, insulin is injected with a syringe or delivered subcutaneously by an external infusion pump. Excessive insulin delivery, however, can result in acute hypoglycemia, which can result in severe bodily injury and/or death. The failure to administer an appropriate amount of insulin to a person with diabetes, however, results in hyperglycemia, which can also result in severe bodily injury and/or death. Between the two conditions of hypoglycemia and hyperglycemia, hypoglycemia is more dangerous. Furthermore, dangerous levels of hypoglycemia are closer to normal levels than dangerous levels of hyperglycemia. Because of the risks involved, there is a need for an improved system for providing information regarding blood glucose levels.

Methods and devices for displaying blood glucose information according to the state of the art are for instance described in EP2500049A1, US2015/0227710A1 and US2011/0201911A1.

The present disclosure is not limited to embodiments that solve any disadvantages or that operate only in environments such as those described above. Rather, this background is only provided to illustrate one example of a technology area where some embodiments described in the present disclosure may be practiced.

### SUMMARY OF THE INVENTION

According to the invention, there are provided a method of displaying blood glucose information according to claim 1 and a device for displaying blood glucose information according to claim 8, preferred embodiments being defined in dependent claims 2-7 and 9-14.

### SUMMARY OF THE DISCLOSURE

Medication delivery systems, methods, and devices provided herein include at least a blood glucose monitor and/or monitoring device (e.g., a BGM, a CGM, etc.) and a display device (e.g., a smartphone having an installed app, tablet, personal computer, or wearable device having an installed app, an installed browser executing a browser application, an installed browser or app receiving information from a server, etc.). In some cases, the display device can serve as the primary user interface for providing blood glucose level information to a user. In some cases, the methods, devices, and systems provided herein can include an insulin delivery device (e.g., an insulin pump, a smart insulin pen, a connected dose-capture cap for an insulin pen, etc., and arranged, e.g., in an open-loop configuration, closed-loop configuration, or combinations thereof) in communication with or part of the blood glucose monitoring device and/or the display device. In some cases, the display device may be configured to monitor or otherwise obtain blood glucose levels and display those levels to a user of the device. For example, the display device may present the current blood glucose level on the display device based on a first scale, such as a logarithmic scale. The display device may display historical glucose levels on a second scale, such as a linear scale. In these and other embodiments, the second scale may be based on or anchored to the first scale. For example, the current blood glucose level may be displayed at a location based on a logarithmic scale along the vertical axis of the display, and the historical blood glucose levels may be displayed as a continuous line starting at the current blood glucose level using a linear scale. Thus, the display device may display the current blood glucose level according to a first scale and the historical blood glucose levels according to a second scale.

In some cases, the display device can project a future blood glucose level. The projected future blood glucose levels can also be displayed according to the second scale and anchored to the current blood glucose level presented based on the first scale. For example, the historic, current, and projected future blood glucose levels may all be displayed on a continuous line according to a linear scale. The location of the line along the vertical axis of the display device may be anchored to the location of the current blood glucose level based on a logarithmic scale.

One or more embodiments of the present disclosure may include a system for displaying blood glucose information that includes a blood glucose monitoring device configured to monitor blood glucose levels of a user, and a display. The system may also include one or more processors, and a non-transitory computer-readable medium containing instructions that, when executed by the one or more processors, cause the system to perform operations. The operations may include obtaining the blood glucose levels from the blood glucose monitoring device, where the blood glucose levels include at least a current blood glucose level and a historic blood glucose level. The operations may also include presenting the current blood glucose level at a first location on the display based on a first scale, and presenting the historic blood glucose level at a second location on the display based on a second scale different from the first scale.

One or more embodiments of the present disclosure may include a method of displaying blood glucose information. The method may include monitoring blood glucose levels of a user, where the blood glucose levels include at least a current blood glucose level and a historic blood glucose level. The method may also include presenting the current blood glucose level using a point indicator along an approximately horizontally centered axis based on a non-linear scale, and presenting the historic blood glucose level and a predicted future blood glucose level on the display as a single smoothed curve passing the point indicator.

The details of one or more implementations of various embodiments are set forth in the accompanying drawings and the description below. Other features, objects, and advantages of the various embodiments will be apparent from the description and drawings, and from the claims.

It is to be understood that both the foregoing general description and the following detailed description are merely examples and explanatory and are not restrictive.

### BRIEF DESCRIPTION OF THE DRAWINGS

Example embodiments will be described and explained with additional specificity and detail through the use of the accompanying drawings in which:
FIG. 1 provides an example system to display blood glucose information using multiple scales;
FIG. 2 is an example display of blood glucose information using multiple scales;
FIGS. 3A and 3B are other example displays of blood glucose information using multiple scales;
FIG. 4 is an additional example display of blood glucose information using multiple scales;
FIG. 5 is another example display of blood glucose information using multiple scales;
FIGS. 6A and 6B illustrate example features of a display of blood glucose information; and
FIG. 7 illustrates a flowchart of an example method of displaying blood glucose information using multiple scales.

### MODE(S) FOR CARRYING OUT THE INVENTION

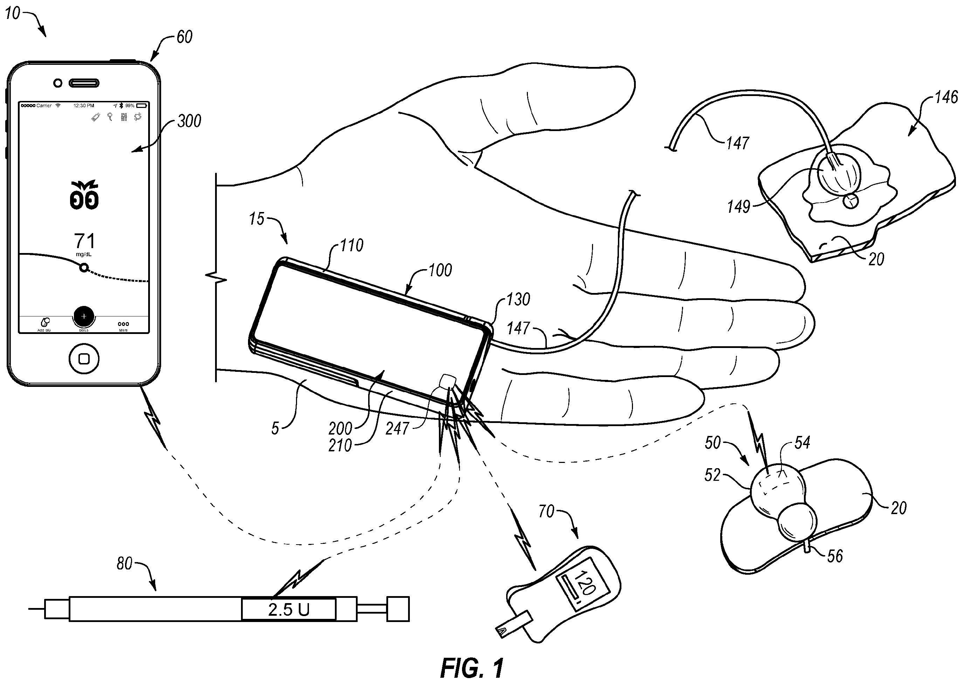

FIG. 1 provides an example system to display blood glucose information using multiple scales, in accordance with one or more embodiments of the present disclosure. The system of FIG. 1 may be described as a diabetes management system 10. The system 10 may include a pump assembly 15 for providing insulin and a continuous glucose monitor 50. As shown, the continuous glucose monitor 50 is in wireless communication with pump assembly 15. In some cases, a continuous glucose monitor can be in wired communication with pump assembly 15. In some cases not shown, a continuous glucose monitor can be incorporated into an insulin pump assembly. As shown, pump assembly 15 can include a reusable pump controller 200 that forms part of the pump assembly 15. In some cases, reusable pump controller 200 is adapted to determine one or more basal delivery rates. In some cases, continuous glucose monitor 50 can act as a controller adapted to communicate basal delivery rates to pump assembly 15.

Pump assembly 15, as shown, can include reusable pump controller 200 and a disposable pump 100, which can contain a reservoir for retaining insulin. A drive system for pushing insulin out of the reservoir can be included in either the disposable pump 100 or the reusable pump controller 200 in a controller housing 210. Reusable pump controller 200 can include a wireless communication device 247, which can be adapted to communicate with a wireless communication device 54 of continuous glucose monitor 50 and other diabetes devices in the system, such as those discussed below. In some cases, pump assembly 15 can be sized to fit within a palm of a hand 5. Pump assembly 15 can include an infusion set 146. Infusion set 146 can include a flexible tube 147 that extends from the disposable pump 100 to a subcutaneous cannula 149 that may be retained by a skin adhesive patch (not shown) that secures the subcutaneous cannula 149 to the infusion site. The skin adhesive patch can retain the cannula 149 in fluid communication with the tissue or vasculature of the PWD so that the medicine dispensed through tube 147 passes through the cannula 149 and into the PWD's body. A cap device 130 can provide fluid communication between an output end of an insulin cartridge (not shown) and tube 147 of infusion set 146. Although pump assembly 15 is depicted as a two-part insulin pump, one piece insulin pumps are also contemplated. Additionally, insulin pump assemblies used in methods and systems provided herein can alternatively be a patch pump.

Continuous glucose monitor 50 (e.g., a glucose monitoring device) can include a housing 52, a wireless communication device 54, and a sensor shaft 56. The wireless communication device 54 can be contained within the housing 52 and the sensor shaft 56 can extend outward from the housing 52. In use, the sensor shaft 56 can penetrate the skin 20 of a user to make measurements indicative of the PWD's blood glucose level or the like. In some cases, the sensor shaft 56 can measure glucose or another analyte in interstitial fluid or in another fluid and correlate that to blood glucose levels. In response to the measurements made by the sensor shaft 56, the continuous glucose monitor 50 can employ the wireless communication device 54 to transmit data to a corresponding wireless communication device 247 housed in the pump assembly 15. In some cases, the continuous glucose monitor 50 may include a circuit that permits sensor signals (e.g., data from the sensor shaft 56) to be communicated to the wireless communication device 54. The wireless communication device 54 can transfer the collected data to reusable pump controller 200 (e.g., by wireless communication to the wireless communication device 247). Additionally or alternatively, the system 10 may include another glucose monitoring device that may utilize any of a variety of methods of obtaining information indicative of a PWD's blood glucose levels and transferring that information to reusable pump controller 200. For example, an alternative monitoring device may employ a micropore system in which a laser porator creates tiny holes in the uppermost layer of a PWD's skin, through which interstitial glucose is measured using a patch. In the alternative, the monitoring device can use iontophoretic methods to non-invasively extract interstitial glucose for measurement. In other examples, the monitoring device can include non-invasive detection systems that employ near IR, ultrasound or spectroscopy, and particular implementations of glucose-sensing contact lenses. In other examples, the monitoring device can include detect glucose levels using equilibrium fluorescence detectors (e.g., sensors including a diboronic acid receptor attached to a fluorophore). Furthermore, it should be understood that in some alternative implementations, continuous glucose monitor 50 can be in communication with reusable pump controller 200 or another computing device via a wired connection. In some cases, continuous glucose monitor 50 can be adapted to provide blood glucose measurements for a PWD when in use for the PWD at regular or irregular time intervals. In some cases, continuous glucose monitor 50 can detect blood glucose measurements at least every thirty minutes, at least every fifteen minutes, at least every ten minutes, at least every five minutes, or about every minute. In some cases, continuous glucose monitor 50 can itself determine a basal delivery rate using methods provided herein and communicate that basal rate to the pump assembly 15. In some cases, continuous glucose monitor 50 can transmit blood glucose measurement data to reusable pump controller 200 and reusable pump controller 200 can use methods provided herein to determine a basal delivery rate. In some cases, a remote controller can receive glucose data from continuous glucose monitor 50, determine a basal delivery rate using methods provided herein, and communicate the basal rate to pump assembly 15.

Diabetes management system 10 may optionally include a blood glucose meter 70 (e.g., a glucose sensor). In some cases, blood glucose meter 70 can be in wireless communication with reusable pump controller 200. Blood glucose meter 70 can take a blood glucose measurement using one or more test strips (e.g., blood test strips). A test strip can be inserted into a strip reader portion of the blood glucose meter 70 and then receive the PWD's blood to determine a blood glucose level for the PWD. In some cases, the blood glucose meter 70 is configured to analyze the characteristics of the PWD's blood and communicate (e.g., via a BLUETOOTH^{®} wireless communication connection) the information to reusable pump controller 200. In some cases, a user can manually input a glucose meter reading. The blood glucose meter 70 can be manually operated by a user and may include an output subsystem (e.g., display, speaker) that can provide the user with blood glucose readings that can be subsequently entered into the reusable pump controller 200 or user interface to collect the data from an unconnected BGM into the system. The blood glucose meter 70 may be configured to communicate data (e.g., blood glucose readings) obtained to reusable pump controller 200 and/or other devices, such as a display device 60 (e.g., a control device). Such communication can be over a wired and/or wireless connection, and the data can be used by system 10 for a number of functions (e.g., calibrating the continuous glucose monitor 50, confirming a reading from the continuous glucose monitor 50, determining a more accurate blood glucose reading for a bolus calculation, detecting a blood glucose level when the continuous glucose monitor 50 is malfunctioning).

In some cases, the system 10 can further include a display device 60 that can communicate with the reusable pump controller 200 through a wireless and/or wired connection with the reusable pump controller 200 (e.g., via a BLUETOOTH^{®} wireless communication connection or a near-field communication connection). In some cases, the display device 60 communicates wirelessly with other diabetes devices of system 10. The display device 60 can be any of a variety of appropriate computing devices, such as a smartphone, a tablet computing device, a wearable computing device, a smartwatch, a fitness tracker, a laptop computer, a desktop computer, and/or other appropriate computing devices. In some cases (for example, where the reusable pump controller 200 does not determine a basal delivery rate), the display device 60 can receive and log data from other elements of the system 10 and determine basal delivery rates using any method or approach, for example, selecting a basal delivery rate or a number of basal delivery rates that produce lowest cost function values. In some cases, the basal delivery rate may be based at least in part on projected blood glucose levels. For example, the display device 60 may predict future blood glucose levels based on historical readings, current IOB, expected delivery rate, etc. The display device may project and/or predict future blood glucose levels in any predictive manner, including the example set forth below.

### Example Techniques for Predicting Future Blood Glucose Values

Systems and methods provided herein can use any suitable physiology model to predict future blood glucose values (represented as BGt and γₜ). In some cases, methods and systems provided herein can predict future blood glucose values using past and current carbohydrate, insulin, and blood glucose values.

Systems and methods provided herein can in some cases estimate a first future blood glucose a model. In some cases, blood glucose can be approximated using two determinist Integrating first order plus dead time (FOPDT) models for the effect of carbohydrates and insulin, combined with an autoregressive (AR2) disturbance model. Accordingly, blood glucose (BG) at time (t) can be estimated using the following equation: ${BG}_{t} = {y}_{t} = {BGc}_{t} + {BGi}_{t} + {BGd}_{t} = {G}_{c} {C}_{t} + {G}_{i} {i}_{t} + {G}_{d} {e}^{at}$

From the equation above, the first element may represent the effect on blood glucose due to carbohydrates: ${G}_{c} = \frac{{K}_{c} \left(1-{α}_{c}\right) {B}^{{C}_{dt}}}{\left(1-{α}_{c}B\right) \left(1-B\right)}$ where:
B is the backward shift operator such that *Bγₜ* = *γ*_{*t*-1}, *B*²*γₜ* = *γ*_{*t*-2}, *B^{k}γₜ* = *γₜ₋ₖ*
${k}_{c} = \frac{ISF}{CR}$ is the carb gain (in units of mg/dl/g)
${α}_{c} = {e}^{- \frac{ts}{{τ}_{c}}}$*,* where τ*_{c}* is the carb time constant (for example, approximately 30 minutes), and
where *ts* is the sampling time (for example, a CGM may use a sampling time interval of every 5 minutes),
*c_{dt} = floor*(τ*_{dc}*/*ts*), where τ*_{dc}* is the carb deadtime (for example, approximately 15 minutes)

From the equation above, the second element may represent the effect on blood glucose due to insulin: ${G}_{i} = \frac{{K}_{i} \left(1-{α}_{c}\right) {B}^{{i}_{dt}}}{\left(1-{α}_{i}B\right) \left(1-B\right)}$ where:
*kᵢ = -ISF* is the insulin gain (in units of mg/dl/unit),
${α}_{i} = {e}^{- \frac{ts}{{τ}_{i}}}$, where τ*ᵢ* is the insulin time constant (for example, approximately 120 minutes),
i*_{dt} = floor*(τ*_{di}*/*ts*), where τ*_{di}* is the insulin deadtime (for example, approximately 30 minutes),

From the equation above, the third element may represent the effect on blood glucose due to disturbances (e.g., the AR2 disturbance model): ${G}_{d} {e}^{{a}_{t}}$ and may be based on the following log-transformed AR2 model: $\left(\frac{{BGd}_{t}}{μ *}\right) = {α}_{1} ln \left(\frac{{BGd}_{t}}{μ *}\right) + {α}_{2} ln \left(\frac{{BGd}_{t - 2}}{μ *}\right) + {α}_{t}$ which when rearranged, yields: ${BGd}_{t} = {BGd}_{t - 1}^{{α}_{1}} {BGd}_{t - 2}^{{α}_{2}} {μ}^{∗ \left(1-{α}_{1}-{α}_{2}\right)} {e}^{{α}_{t}}$ where, in some examples, ${a}_{t} ∼ Normal \left(0, {σ}_{a}\right)$ and ${σ}_{a} \approx 50 % ln \left(σ*\right) \sqrt{\frac{1 + {α}_{2}}{1 - {α}_{2}} \left.\left({\left(1-{α}_{2}\right)}^{2}\right)-{α}_{1}^{2}\right)}$ with $μ * ∼ {10}^{Normal \left(2.09, 0.08\right)} and σ * ∼ {10}^{Normal \left(0.15, 0.028\right)}$ such that ${α}_{1} \approx 1.6442 , {α}_{2} \approx 0.6493 .$

Using the above notation, expansion of the initial equation for *BGₜ* may be represented by the equation: ${BG}_{t} = \frac{{k}_{c} \left(1-{α}_{c}\right)}{\left(1-{α}_{c}B\right) \left(1-B\right)} {c}_{t - {dt}_{c}} + \frac{{k}_{i} \left(1-{α}_{i}\right)}{\left(1-{α}_{i}B\right) \left(1-B\right)} {i}_{t - {dt}_{i}} + {BGd}_{t - 1}^{{α}_{1}} {BGd}_{t - 2}^{{α}_{2}} {μ}^{∗ \left(1-{α}_{1}-{α}_{2}\right)}$

Systems and methods provided herein can in some cases calculate an amount of insulin on board (IOB) and/or an amount of carbohydrates on board (COB) in order to predict future blood glucose values. IOB and COB represent the amount of insulin and carbohydrates, respectively, which have been infused and/or consumed but not yet metabolized. Knowledge of IOB and COB can be useful for a user of a method or system provided herein when it comes to bolus decisions to prevent insulin stacking, but knowledge of IOB and COB can also be used in methods and systems provided herein to predict future blood glucose values.

IOB and COB represent the amount of insulin and carbohydrates, respectively, which have been infused and/or consumed but not yet metabolized. Knowledge of IOB can be useful in correcting bolus decisions to prevent insulin stacking. Knowledge of IOB and COB can be useful for predicting and controlling blood glucose. Both insulin infusion and carbohydrate consumption can involve deadtime or transportation delay (e.g., it can take ten to forty minutes for insulin and/or carbohydrates to begin to affect blood glucose). During the period immediately after entering the body (e.g., during the deadtime period), it can be beneficial to account for IOB and COB in any decisions such as bolusing. This can be called "Decision" IOB/COB. "Action" IOB/COB, on the other hand, can represent the insulin and/or carbohydrates available for action on blood glucose. In some cases, Decision IOB can be a displayed JOB, while Action IOB can be an IOB determined for use in selecting a basal delivery rate or profile in methods and systems provided herein.

From the equations above, ${BG}_{it} = \frac{- ISF \left(1-{α}_{i}\right) {B}^{{i}_{dt}}}{\left(1-{α}_{i}B\right) \left(1-B\right)} {i}_{t - {i}_{dt}}$ where ${BY}_{t} = {Y}_{t - 1} , {B}^{2} {Y}_{t} = {Y}_{t - 2} , {B}^{k} {Y}_{t} = {Y}_{t - k}$
${α}_{i} = {e}^{- \frac{ts}{{τ}_{i}}}$ where *τᵢ* is the insulin time constant (for example, approximately 120 minutes)
*i_{dt} =* floor(*τ_{di}*/*ts*), where *τ_{di}* is the insulin deadtime (for example, approximately 30 minutes) and where *ts* is the sampling time (for example, a CGM may use a sampling time interval of every 5 minutes)

### "Decision" IOB

In some embodiments, Decision IOB at time (t) (*IOB_Dₜ*) may be calculated according to the following mathematical process: $IOB _ {D}_{t} = IOB _ {D}_{t - 1} - \frac{{BGi}_{t} - {BGi}_{t - 1}}{- ISF} + {i}_{t}$ or, alternatively, $∇ IOB _ {D}_{t} = - \frac{∇ {BGi}_{t}}{- ISF} + {i}_{t}$ substituting the equation above for *BGᵢₜ* into the equation for *IOB_Dₜ* or ∇*IOB_Dₜ* yields ${IOB}_{{D}_{t}} = \frac{1 - {α}_{i} B - \left(1-{α}_{i}\right) {B}^{i} dt}{1 - \left({α}_{i}+1\right) B + {α}_{i} {B}^{2}} {i}_{t}$ or, alternatively, $∇ IOB _ {D}_{t} = - \frac{1 - {α}_{i}}{1 - {α}_{i} B} {i}_{t - {i}_{dt}} + {i}_{t}$

### "Action" IOB

In some embodiments, Action IOB at time (t) (*IOB_Aₜ*) may be calculated according to the following mathematical process: $IOB _ {A}_{t} = \frac{1}{1 - {α}_{i} B} {i}_{t - {i}_{dt}}$

For an arbitrary series of insulin infusions, using an infinite series of expansions of $\frac{1}{1 - {α}_{i} B}$, *IOB_Aₜ* may be represented by ${IOB}_{{A}_{t}} = {\sum }_{k = 0}^{n} {α}_{i}^{k} {i}_{t - k - {i}_{dt}}$

Stated another way, ${BGi}_{t} = \frac{- ISF \left(1-{α}_{i}\right)}{1 - B} IOB _ {A}_{t}$

The formulas for COB, including Action COB and Decision COB, may be developed in a similar fashion, using the equation above related to*G_{c}*: ${G}_{ct} = \frac{{k}_{c} \left(1-{α}_{c}\right) {B}^{{c}_{dt}}}{\left(1-{α}_{c}B\right) \left(1-B\right)}$

Accordingly, future blood glucose data can be estimated using current or recent blood glucose data, data about when carbohydrates were consumed, and/or data regarding when insulin was and/or will be administered. Moreover, because evaluated insulin delivery profiles and/or rates include basal insulin delivery rates above and below the BBR, those insulin delivery rates above BBR can be added to the IOB calculation and insulin delivery rates below the BBR can be subtracted from the IOB. In some cases, a variation in a Decision IOB due to actual variations from BBR can be limited to positive deviations in order to prevent a user from entering an excessive bolus.

In some cases, a user can input relevant data into the display device 60. In some cases, the display device 60 can be used to transfer data from the reusable pump controller 200 to another computing device (e.g., a back-end server or a cloud-based device). In some cases, the display device 60 provides a user interface (e.g., graphical user interface (GUI), speech-based user interface, motion-controlled user interface) through which users can provide information to control operation of the reusable pump controller 200 and the system 10. For example, the display device 60 can be a mobile computing device running a mobile app that communicates with reusable pump controller 200 over short-range wireless connections (e.g., BLUETOOTH^{®} connection, Wi-Fi Direct connection, near-field communication connection, etc.) to provide status information for the system 10 and allow a user to control operation of the system 10 (e.g., toggle between delivery modes, adjust settings, log food intake, change a fear of hypoglycemia index (FHI), confirm/modify/cancel bolus dosages, and the like).

By way of further characterization, a GUI typically includes one or more information regions and active/activatable regions. As used in this disclosure, an information region is a region of a GUI which presents information to a user. An activatable region is a region of a GUI, such as a button, slider, or a menu, which allows the user to take some action with respect to the GUI (e.g., if manipulated, such as with a point-and-click interface, a touch interface, an audio interface, etc.). Some information regions are also activatable regions in that they present information and enable some action that may be taken by a user. Activatable regions may be displayed as GUI elements/objects, for example, buttons, sliders, selectable panes, menus, etc., all of various shapes and sizes.

Generally, if an interaction is detected by a GUI, a process is used to determine the activatable regions of the GUI to which the contact corresponds, if any. For example, if a touch is detected at an "ENTER", then responsive to the detected touch a process may determine that the contact was at the ENTER button. The ENTER button is an activatable region, so one or more events may be created at the GUI and/or an underlying application that invoked the GUI.

Optionally, system 10 may include a bolus administering device 80 (e.g., a syringe, an insulin pen, a smart syringe with device communication capabilities, or the like) through which bolus dosages can be manually administered to a PWD. In some cases, a suggested dosage for a bolus to be administered using the bolus administering device 80 can be output to a user via the user interface of reusable pump controller 200 and/or the user interface of the display device 60. In some cases, the bolus administering device 80 can communicate through a wired and/or wireless connection with reusable pump controller 200 and/or the display device 60. In some cases, system 10 can allow users to input insulin deliveries made using a syringe or insulin pen.

In some cases, the display device 60 includes a display 300. The display device 60 can display blood glucose information. For example, the display device 60 may obtain blood glucose readings from the CGM 50 or the blood glucose meter 70. The display device 60 may store the blood glucose readings as historic blood glucose readings and the most recent blood glucose level as the current blood glucose level. The display device 60 may display various aspects of the blood glucose levels and/or projected blood glucose levels as explained in greater detail below.

While one embodiment of a diabetes management system is illustrated in FIG. 1, it will be appreciated that any number, type, or style of diabetes management devices may be utilized in conjunction with the present disclosure. For example, a patch pump, a syringe, etc., may be utilized to enter doses of insulin delivered to a PWD. As another example, any blood glucose reading device may be utilized, such as a BGM, a CGM, a flash glucose monitor (FGM), or any other blood glucose reading device. In some embodiments, an insulin delivery device may not be used.

Modifications, additions, or omissions may be made to FIG. 1 without departing from the scope of the present disclosure. For example, the system 10 may include any type or style of insulin delivery devices and/or monitoring devices. As another example, the display device 60 may take any form or style of computing device. As an additional example, the display device 60 may be coupled with a remote cloud device (not illustrated) that may store one or more aspects of the monitored and/or projected blood glucose levels and/or insulin delivery rates and/or projections. Such a cloud device may be accessible by a third party (e.g., a physician) or a PWD.

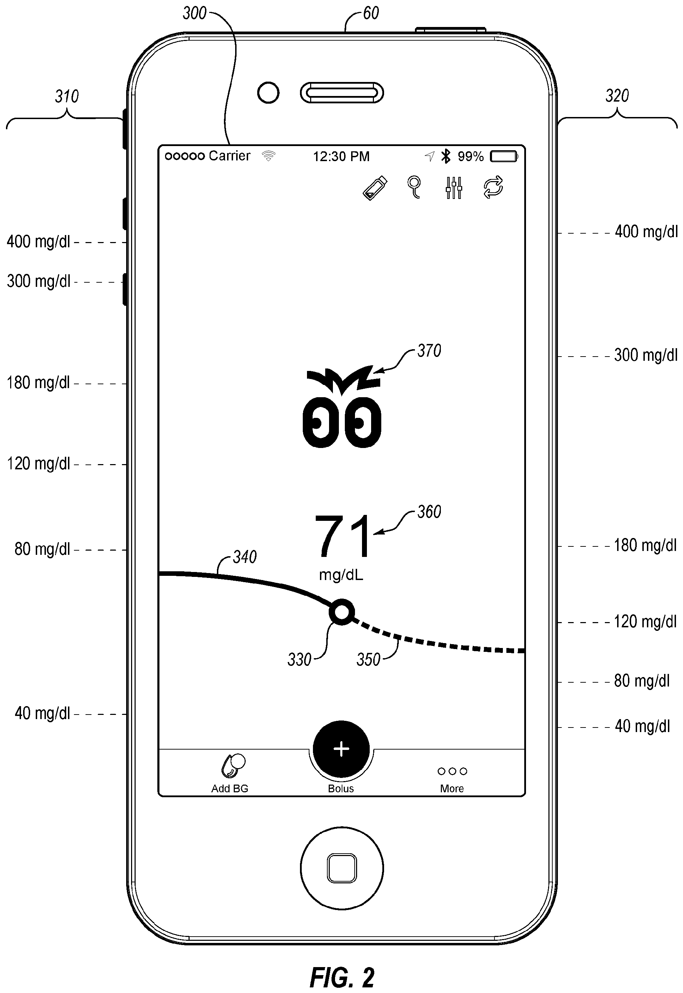

FIG. 2 is an example display 300 of blood glucose information using multiple scales, in accordance with one or more embodiments of the present disclosure. The display 300 may be part of the display device 60 of FIG. 1. The display 300 may present blood glucose information based on a first scale 310 and a second scale 320. In some cases, the display 300 may present a current blood glucose level 330, historic blood glucose levels 340, and projected blood glucose levels 350. Additionally or alternatively, the display 300 may include a numerical representation 360 of the current blood glucose level 330 and/or an avatar 370 for a glanceable view of blood glucose information.

In some embodiments, the blood glucose information may be displayed on the display 300 using both the first scale 310 and the second scale 320. As illustrated in FIG. 2, the first scale 310 may be a risk-based scale such that areas of greater risk may receive more of the total space of the display 300. For example, the risk of blood glucose levels of a PWD going below normal blood glucose levels (e.g., hypoglycemia) may be more severe than the risks of a PWD going above normal blood glucose levels (e.g., hyperglycemia). Using a risk-based scale, blood glucose levels corresponding to hypoglycemia may cover a larger portion of the display 300 than other scales.

The first scale 310 may include a logarithmic scale such that lower values receive logarithmically more space of the display 300. For example, as illustrated in FIG. 2, according to the first scale 310, the range from 400 mg/dL to 180 mg/dL may cover approximately 35% of the display 300 used for blood glucose information, the range from 180 mg/dL to 80 mg/dL may cover approximately 35% of the display 300 used for blood glucose information, and the range from 80 mg/dL to 40 mg/dL may cover approximately 30% of the display 300 used for blood glucose information.

The second scale 320 may illustrate a linear scale that also covers the display 300 used for displaying blood glucose information. For example, as illustrated in FIG. 2, according to the second scale 320, the range from 400 mg/dL to 180 mg/dL covers approximately 61% of the display 300 used for blood glucose information, the range from 180 mg/dL to 80 mg/dL covers approximately 28% of the display 300 used for blood glucose information, and the range from 80 mg/dL to 40 mg/dL covers approximately 11% of the display 300 used for blood glucose information. In these and other embodiments, the second scale 320 may provide a framework within which various aspects of blood glucose information may be displayed. Intuitively, some users such as PWDs may more readily understand and relate to a linear scale (such as the second scale 320) as opposed to a logarithmic scale (such as the first scale 310).

In some embodiments, the current blood glucose level 330 may be displayed based on the first scale 310. For example, an icon, marker, or other indication of the current blood glucose level 330 may be displayed along a vertical axis of the display 300 based on the logarithmic first scale 310. In some embodiments, the current blood glucose level 330 may be displayed at approximately the center of the display 300 along a horizontal axis of the display 300. Additionally or alternatively, the current blood glucose level 330 may move along a vertical axis along the center of the horizontal axis as changes occur in the current blood glucose level 330.

In some cases, the current blood glucose level 330 may be based on a BGM, a CGM, an FGM, or any other blood glucose monitoring device. In these and other cases, "current" blood glucose levels may include the most recent blood glucose reading, a blood glucose reading within a time threshold (e.g., within the last 1 minute, within the last 2 minutes, within the last 5 minutes, within the last 10 minutes, within the last 15 minutes, etc.), or combinations thereof. In some embodiments, an icon representing the current blood glucose level 330 may change based on how recent the latest reading has been taken. For example, the color of the icon may fade over time, or the icon may no longer be present after a certain duration of time, etc.

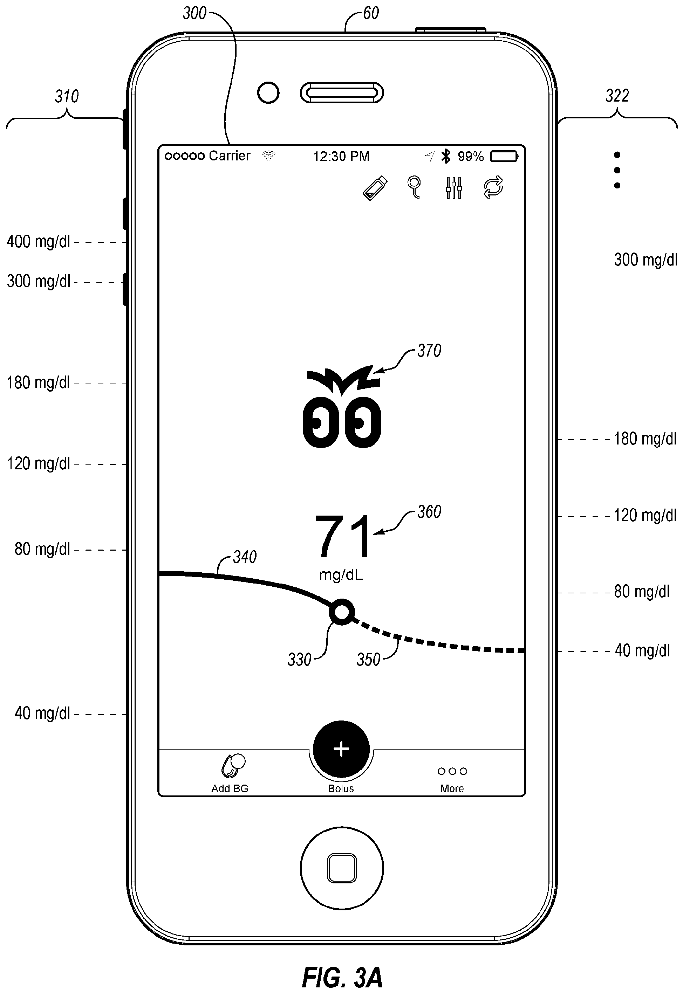

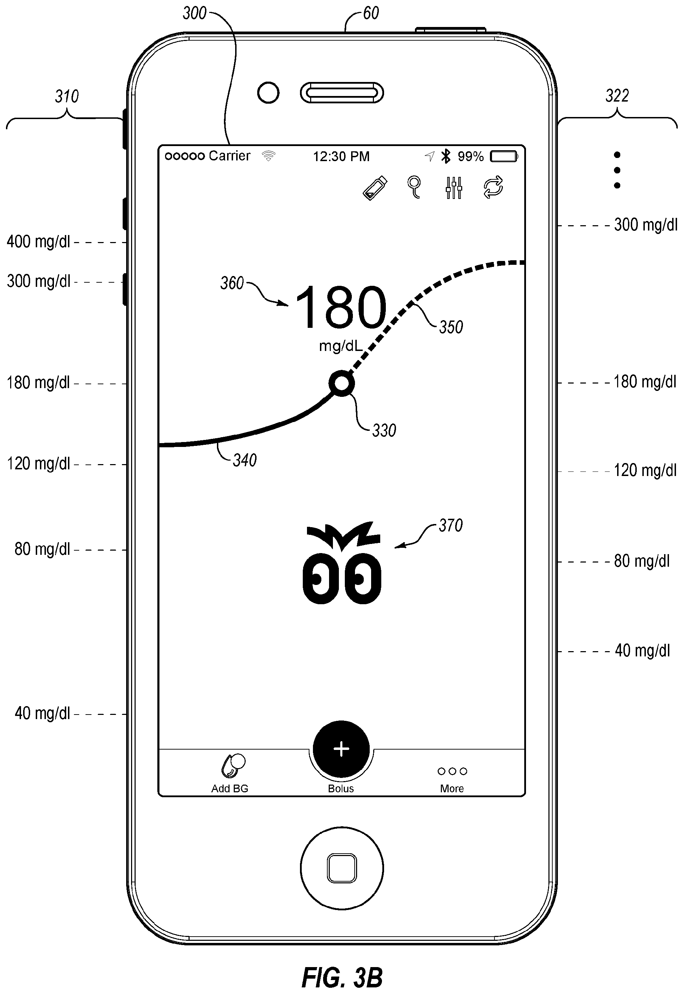

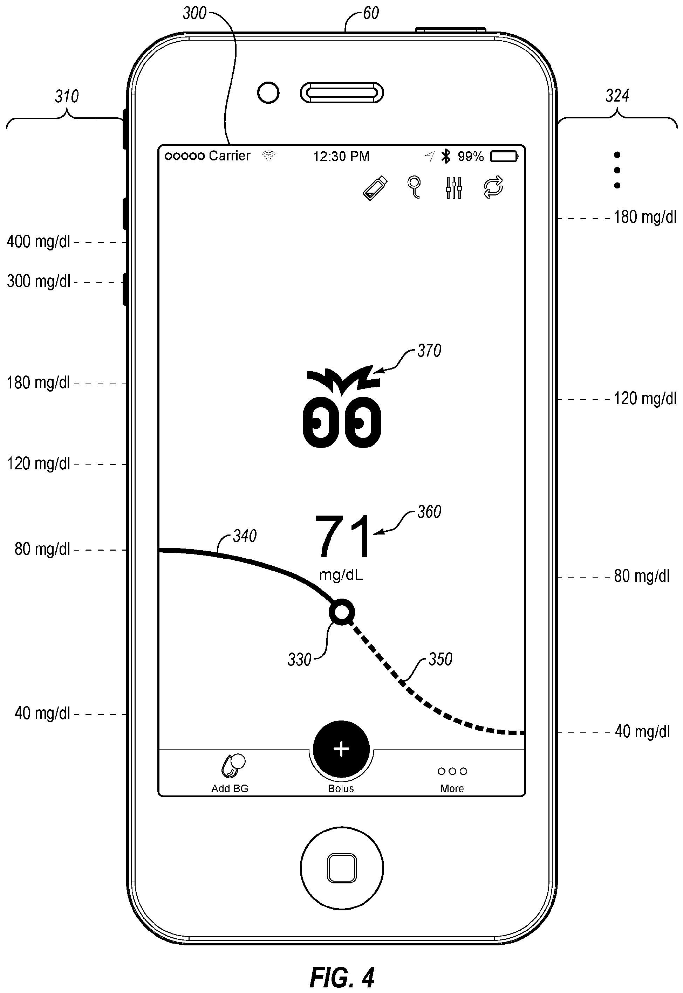

In some embodiments, the historic blood glucose levels 340 and/or the projected blood glucose levels 350 may be displayed according to the second scale 320. In some embodiments, the second scale 320 may be anchored or oriented based on the first scale 310. Examples, of such anchoring may be explained in greater detail with reference to FIGS. 3 and/or 4. The second scale 320 may be displayed in FIG. 2 to illustrate variations in the amount of the display 300 covered by various spans of blood glucose levels according to the first scale 310 and the second scale 320.

By displaying blood glucose information using the first scale 310, a greater visual distinction is observable for hypoglycemic ranges as compared to the second scale 320. Furthermore, using the first scale 310, the range of 300-400 mg/dL (which is rarely used) only uses approximately 10% of the display 300 rather than over 25% of the display 300 when using the second scale 320.

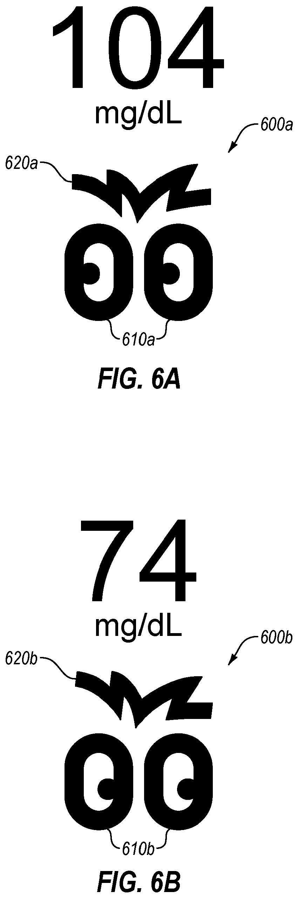

The avatar 370 for glanceability may be described with greater detail with reference to FIGS. 6A and/or 6B.

FIGS. 3A and 3B are other example displays 300 of blood glucose information using multiple scales, in accordance with one or more embodiments of the present disclosure. FIGS. 3A and 3B are similar to FIG. 2, with the variation that a second scale 322 is anchored differently than in FIG. 2.

As illustrated in FIGS. 3A and 3B, the second scale 322 may be anchored along the vertical axis of the display 300 based on the location of the current blood glucose level 330 based on the first scale 310. For example, the current blood glucose level 330 illustrated in FIG. 3A is approximately 71 mg/dL. The second scale 322 may be shifted along the vertical axis of the display 300 such that the current blood glucose level 330, located along the vertical axis in the first scale 310, is also at the same vertical position along the vertical axis of the display 300. The second scale 322 may be anchored in the vertical axis based on the current blood glucose level 330 according to the first scale 310. As another example, as illustrated in FIG. 3B, the current blood glucose level 330 is approximately 180 mg/dL, and the second scale 322 is anchored to that value according to the first scale 310.

As illustrated in FIG. 3A, the current blood glucose level 330 of 71 mg/dL may be displayed along the vertical axis of the display 300 according to the first scale 310. The historical blood glucose levels 340 may be displayed relative to the current blood glucose level 330 according to the second scale 322. For example, a continuous line from 71 mg/dL may proceed back in time up to approximately 85 mg/dL on the second scale 322 (while if the first scale 310 were used the historical blood glucose levels 340 would indicate only approximately 75 mg/dL). As another example, a continuous line from 71 mg/dL may proceed forward in time down to approximately 40 mg/dL for the projected blood glucose levels 350 according to the second scale 322 (while if the first scale 310 were used the projected blood glucose levels 350 would only be approximately 55 mg/dL). In one embodiment, the continuous line may comprise visual indicators to show that a part of the continuous line corresponds to historical, current, and projected blood glucose levels. For example, in FIG. 3A, historical blood glucose levels 340 are shown as a solid line, current blood glucose levels 330 is shown as circle that defines a white space, and projected blood glucose levels 350 are shown as a dashed line.

In some cases, the historical blood glucose levels 340 may be smoothed in the display 300. For example, the historical blood glucose levels 340 may follow a line that is smoothed to follow a curved, rather than a jagged line. Additionally or alternatively, extrapolation may be performed on blood glucose levels in between points of actual readings to facilitate smoothing of the historical blood glucose levels 340.

In some cases, the historical blood glucose levels 340 may be corrected based on an updated and/or corrected current blood glucose level 330. For example, if a newly calibrated blood glucose monitoring device provides a current blood glucose level 330 different than what is expected from the historic readings before the calibration of the device, the historical readings may be corrected based on the updated current blood glucose level 330 to smooth the historical blood glucose levels 340.

Using such an approach, the benefit of the risk-based scale may be utilized in displaying the current blood glucose level 330 along the vertical axis of the display 300. For example, hypoglycemic blood glucose levels have a larger area of the display 300 than with a linear scale such that a PWD will have a greater visual cue of how close to hypoglycemic levels they are approaching. Additionally, by using the second scale 322 for the historic blood glucose levels 340 and/or the projected blood glucose levels 350, a PWD may be able to observe trends according to a scale with which they are more familiar or comfortable and/or that may be more intuitive to the PWD.

In some cases, the future blood glucose levels 350 and/or the historic blood glucose levels 340 may be displayed as a sparkline. For example, the future blood glucose levels 350 may be displayed without a specific scale or without a specific numerical indication of the future blood glucose levels 350. Using such an embodiment, a PWD may be provided with a sense of the direction of their future blood glucose levels 350 without being overly focused on the exact levels. Additionally or alternatively, such a sparkline may be smoothed such that no clear data points are evidenced by a sudden change in direction of the line, but rather illustrate trends. Such a sparkline may provide glanceability where a PWD may glance at the display 300 and recognize generally what their historic blood glucose levels have been (e.g., low/high) and what their predicted blood glucose levels are expected to be (e.g., trending low/high) without being overloaded with data.

In some cases, an interface of the display 300 may be invoked to view a more detailed graph of the blood glucose levels conveyed by the sparkline. For example, a PWD may swipe down on the sparkline to view a more detailed graph that includes specific data points, axis labels, numerical indicators, reference lines, trend lines, and/or any other graph feature to provide a PWD a more detailed view into historic, current, and/or future blood glucose levels.

In some cases, a background color or colors may be utilized to improve glanceability. For example, a region above the line and/or a region below the line of the historic blood glucose levels 340, current blood glucose level 330, and future blood glucose levels 350 may vary based on blood glucose levels. Such an approach can give a PWD a rapid indication of blood glucose levels. For example, the color and/or color change can be based on a threshold (e.g., 180 mg/dL (or 200 or 250 or 300) for hyperglycemic blood glucose levels and 80 mg/dL (or 70 or 50 or 40) for hypoglycemic blood glucose levels). In some cases, the color may be based on the current blood glucose level 330. Additionally or alternatively, the color and/or color change may be based on future blood glucose levels 350. In some cases the region below the line may be based on the current blood glucose levels 330 and the region above the line may be based on the future blood glucose levels 350, or vice versa. In some cases, the color change can be an instant color change, or can based on a gradient shift as the blood glucose level changes. For example, if a PWD has a current blood glucose level of 100 mg/dL the color may be gray, and as the current blood glucose level drops below 80 mg/dL, the color may change from gray to blue. As another example, if the current blood glucose level is 160 mg/dL with a projected future blood glucose level trending up to 250 mg/dL, the color may be gray and transitioning to red such that as the projected blood glucose levels cross the threshold, the color is red. In some cases, the color yellow can be used to indicate hyperglycemia, blue can be used to indicate euglycemia, and red can be used to indicate hypoglycemia. Any color scheme may be used.

FIG. 4 is an additional example display 300 of blood glucose information using multiple scales, in accordance with one or more embodiments of the present disclosure. FIG. 4 is similar to FIGS. 3A and 3B except the second scale 324 of FIG. 4 is made larger than the second scale 322 of FIGS. 3A and 3B, while still being a linear scale and still anchored based on the first scale 310.

As illustrated in FIG. 4, the second scale 324 may be made larger such that changes in blood glucose levels and trends thereof may be more readily observed. For example, the historic blood glucose levels 340, the current blood glucose level 330, and the future blood glucose levels 350 may span from 85 mg/dL to 40 mg/dL and cover approximately 25% of the space of the display 300 based on the second scale 324. In FIGS. 3A and 3B, the same range covers approximately 12% of the display 300.

Modifications, additions, or omissions may be made to FIGS. 2-4 without departing from the scope of the present disclosure. For example, the display 300 may include numerical markings along the vertical axis of the display indicating the first scale and/or the second scale. As another example, the first scale may be any scale to emphasize certain regions of potential blood glucose levels. As an additional example, the second scale may be any magnification of the linear scale.

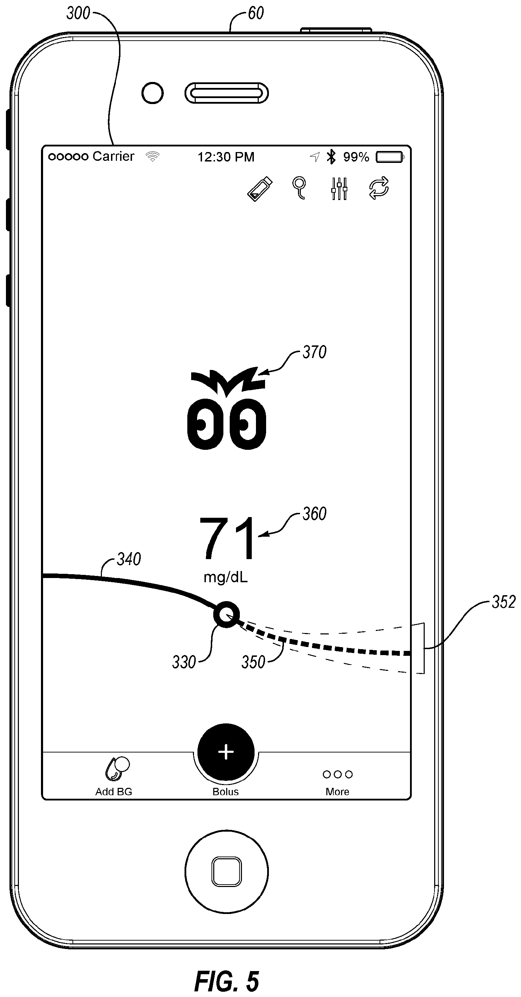

FIG. 5 is another example display 300 of blood glucose information using multiple scales. As illustrated in FIG. 5, in some embodiments, the projected blood glucose levels 350 may include a feature to illustrate a level of confidence in the projected blood glucose levels 350.

Depending on the approach used to project the future blood glucose levels, different future blood glucose levels may have different levels of confidence in the accuracy of the projected blood glucose levels. In some embodiments, as the projected blood glucose levels go further into the future, the confidence levels may decrease (e.g., the accuracy of the projected blood glucose level may be less and less sure).

In some embodiments, the level of confidence may be illustrated by a range 352 of potential future blood glucose levels. For example, the range 352 may illustrate that the projected future blood glucose levels close to the current blood glucose level 330 may be only a small range of values, and as the projected blood glucose levels go further into the future, the range 352 increases. The level of confidence may be illustrated in any manner, such as by error bars, maximum and/or minimum values, color changes, transparency, etc.

Modifications, additions, or omissions may be made to FIG. 5 without departing from the scope of the present disclosure. For example, the display 300 may include any approach to display confidence levels of the projected blood glucose levels.

FIGS. 6A and 6B illustrate example features of a display of blood glucose information, in accordance with one or more embodiments of the present disclosure. For example, FIG. 6A illustrates a first avatar 600a and FIG. 6B illustrates a second avatar 600b. The first avatar 600a and the second avatar 600b may correspond to the same PWD but at different points in time.

The avatar 600a may include features to facilitate glanceability (which may be further characterized as "glanceable," and means understandable at a glance or with occasional glances) regarding blood glucose information and/or operation of a diabetes management system (such as the system 10 of FIG. 1). In some embodiments, the avatar 600a may include a facial avatar with various features of the avatar 600a associated with various blood glucose information and/or aspects of the operation of the diabetes management system.

In some embodiments, the eyes 610a may represent the current blood glucose information. For example, whether the pupils of the eyes 610a are on the left or the right of the eyeballs may indicate whether the current blood glucose level is above or below a threshold. As another example, the pupils of the eyes 610a may be at an approximate location along the vertical length of the eyeball correlated with the vertical length of a display (such as the display 300).

In some embodiments, the eyebrows 620a may represent trends of historical and/or projected blood glucose levels. For example, if the historical blood glucose levels were higher than the current blood glucose level, the left side of the eyebrows 620a may be tilted in an upward direction and if the historical blood glucose levels were lower than the current blood glucose level, the left side of the eyebrows 620a may be tilted in a downward or lower direction. As another example, if the trend of the projected blood glucose levels are approximately the same as the current blood glucose levels, the right side of the eyebrows 620a may be relatively level. Alternatively, if the trend of the projected blood glucose levels are higher than the current blood glucose level, the right side of the eyebrows 620a may be curved upwards.

In some embodiments, a color of the avatar may shift and/or change based on blood glucose information and/or operation of the diabetes management system. For example, if the PWD is projected to have low blood sugar level below a threshold or the current blood glucose level is below a threshold, the coloration of the avatar may change to a warning color such as red. As another example, if the PWD is trending toward higher blood glucose levels, the tone of the color of the avatar 600a may get lighter and if trending toward lower blood glucose levels, the tone of the color of the avatar 600a may get darker. In some cases, the color change may be a background shading of the avatar 600a and may change in a similar manner as described for the background color. For example, the color, shade, etc., of the avatar 600a may change based on whether a blood glucose level is hyperglycemic, euglycemic, or hypoglycemic.

FIG. 6B provides an illustration of changes from the avatar 600a to avatar 600b as the blood glucose level transitions from 104 mg/dL to 74 mg/dL. For example, eyebrows 620b of the avatar 600b are raised on the left, indicating that the historical values (e.g., 104 mg/dL) are higher than the present value. As another example, the pupils of the eyes 610b may transition to the right side of the eyeball as the blood glucose level may have dropped below a threshold value.

While described in terms of blood glucose information, any aspect of blood glucose information and/or operation of the diabetes management system is contemplated. For example, a feature of the avatar 600a may correspond to whether or not the diabetes management system is providing personalized basal insulin delivery. As another example, a feature of the avatar may be related to whether or not there is an error or malfunction in the diabetes management system 10, such as an occlusion in an insulin pump.

Modifications, additions, or omissions may be made to FIGS. 6A and 6B without departing from the scope of the present disclosure. For example, the avatars 600a and/or 600b may be correlated with any aspect of blood glucose information and/or any aspect of operation of the diabetes management system. As another example, while illustrated as a facial avatar, any multi-component image or avatar may be used.

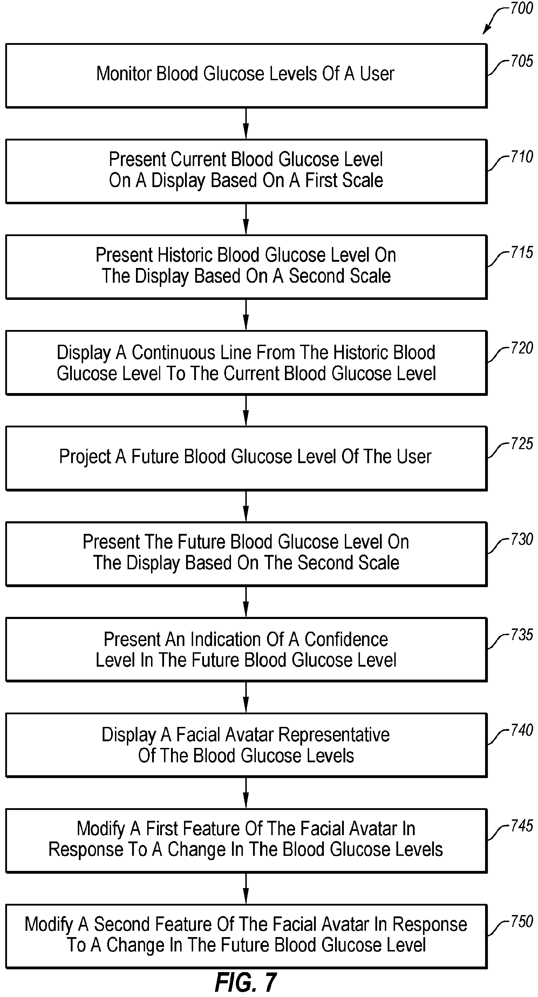

FIG. 7 illustrates a flow diagram of an example method 700 of presenting blood glucose information using multiple scales. The method 700 may be performed by any suitable system, apparatus, or device. For example, the system 10, the pump assembly 15, the display device 60 of FIG. 1, and/or a remote server may perform one or more of the operations associated with the method 700. Although illustrated with discrete blocks, the steps and operations associated with one or more of the blocks of the method 700 may be divided into additional blocks, combined into fewer blocks, or eliminated, depending on the desired implementation.

At block 705, blood glucose levels may be monitored for a PWD. For example, a diabetes management system (such as the system 10 of FIG. 1) may monitor the blood glucose levels using a CGM and/or a BGM. In these and other embodiments, a single device may monitor the blood glucose levels or a separate device may obtain the blood glucose levels from a monitoring device.

At block 710, a current blood glucose level may be presented on a display based on a first scale. For example, the current blood glucose level may be displayed at a first location along the vertical axis of the display based on a first scale, such as a risk-based scale. The risk-based scale may include a logarithmic scale.

At block 715, historic blood glucose levels may be presented on the display based on a second scale. For example, the historic blood glucose levels may be displayed at a second location based on the second scale and the location of the current blood glucose level. For example, the second scale may be anchored to the location of the current blood glucose level. The second scale may include a linear scale.

At block 720, a continuous line may be displayed from the current blood glucose level to one or more of the historic blood glucose levels. For example, a line may be extrapolated between the current blood glucose level and a historic level such that a continuous line may be displayed.

At block 725, a future blood glucose level of the user may be projected.

At block 730, the projected future blood glucose level of the block 725 may be presented on the display based on the second scale. For example, the future blood glucose level may be presented according to the same scale as the historic blood glucose levels. Additionally or alternatively, a continuous line may be displayed connecting the future blood glucose level and the current blood glucose level.

At block 735, an indication of a confidence level in the future blood glucose level may be presented.

At block 740, a facial avatar representative of the blood glucose levels may be displayed. Additionally or alternatively, another type of avatar or other multi-feature image may be displayed.

At block 745, a first feature of the facial avatar may be modified in response to a change in the blood glucose levels. For example, based on a newly received blood glucose level the first feature may be modified. As another example, the first feature may be modified based on changes to trends in blood glucose levels (e.g., based on historical blood glucose levels).

At block 750, a second feature of the facial avatar may be modified in response to a change in the future blood glucose level. For example, the second feature may be modified based on a trend associated with the future blood glucose level or a projected blood glucose level.

Modifications, additions, or omissions may be made to the method 700 without departing from the scope of the present disclosure. For example, the operations of the method 700 may be implemented in differing order. Additionally or alternatively, two or more operations may be performed at the same time. Furthermore, the outlined operations and actions are provided as examples, and some of the operations and actions may be optional, combined into fewer operations and actions, or expanded into additional operations and actions without detracting from the essence of the disclosed embodiments.

The embodiments described herein may include the use of a special-purpose or general-purpose computer including various computer hardware or software modules, as discussed in greater detail below.

Embodiments described herein may be implemented using computer-readable media for carrying or having computer-executable instructions or data structures stored thereon. Such computer-readable media may be any available media that may be accessed by a general-purpose or special-purpose computer. By way of example, and not limitation, such computer-readable media may include non-transitory computer-readable storage media including Random-Access Memory (RAM), Read-Only Memory (ROM), Electrically Erasable Programmable Read-Only Memory (EEPROM), Compact Disc Read-Only Memory (CD-ROM) or other optical disc storage, magnetic disk storage or other magnetic storage devices, Flash memory devices (e.g., solid state memory devices), or any other storage medium that may be used to carry or store desired program code in the form of computer-executable instructions or data structures and which may be accessed by a general-purpose or special-purpose computer. Combinations of the above may also be included within the scope of computer-readable media.

Computer-executable instructions comprise, for example, instructions and data which cause a general-purpose computer, special-purpose computer, or special-purpose processing device (e.g., one or more processors) to perform a certain function or group of functions. Although the subject matter has been described in language specific to structural features and/or methodological acts, it is to be understood that the subject matter is not necessarily limited to the specific features or acts described above. Rather, the specific features and acts described above are disclosed as examples.

As used herein, the terms "module" or "component" may refer to specific hardware implementations configured to perform the operations of the module or component and/or software objects or software routines that may be stored on and/or executed by general-purpose hardware (e.g., computer-readable media, processing devices, etc.) of the computing system. In some embodiments, the different components, modules, engines, and services described herein may be implemented as objects or processes that execute on the computing system (e.g., as separate threads). Routines and operations performed by a processor, machine-readable instructions that a processor executes to perform such routines and operations, and functions that such routines and operations enable, may be described herein as an "algorithm" or a number of related "algorithms." While some of the system and methods described herein are generally described as being implemented in software (stored on and/or executed by general-purpose hardware), specific hardware implementations or a combination of software and specific hardware implementations are also possible and contemplated. In the present description, a "computing entity" may be any computing system as previously defined herein, or any module or combination of modulates running on a computing system.

Any ranges expressed herein (including in the claims) are considered to be given their broadest possible interpretation. For example, unless explicitly mentioned otherwise, ranges are to include their end points (e.g., a range of "between X and Y" would include X and Y). Additionally, ranges described using the terms "approximately" or "about" are to be understood to be given their broadest meaning consistent with the understanding of those skilled in the art. Additionally, the term approximately includes anything within 10%, or 5%, or within manufacturing or typical tolerances.

All examples and conditional language recited herein are intended for pedagogical objects to aid the reader in understanding the disclosure and the concepts contributed by the inventor to furthering the art and are to be construed as being without limitation to such specifically recited examples and conditions. Although embodiments of the present disclosure have been described in detail, it should be understood that the various changes, substitutions, and alterations could be made hereto, the invention being defined by the appended claims.

## Claims

1. A method of displaying blood glucose information, the method comprising:
monitoring blood glucose levels of a user, the blood glucose levels including at least a current blood glucose level (330) and a historic blood glucose level (340);
determining a first location along a vertical axis of a display (300) for the current blood glucose level (330) by applying a non-linear vertical scale (310) to the current blood glucose level (330), the non-linear vertical scale (310) being a logarithmic scale such that lower blood glucose values are assigned to locations receiving logarithmically more space along the vertical axis of the display (300);
shifting a linear vertical scale (322) of blood glucose levels along the vertical axis of the display (300) based on the first location such that the current blood glucose level (330) corresponds to the first location on the linear vertical scale (322);
determining second locations along the vertical axis of the display (300) for the historic blood glucose level (340) and a predicted future blood glucose (350) level based on the shifted linear vertical scale (322) relative to the first location;
presenting the current blood glucose level (330) on the display (300) at the first location using a point indicator centered on a horizontal axis, wherein the point indicator is displayed according to the non-linear logarithmic scale; and
presenting the historic blood glucose level (340) and a predicted future blood glucose (350) level on the display (300) at the second locations as a single smoothed curve passing through the point indicator, wherein the historic blood glucose level (340) and the predicted future blood glucose (350) are displayed according to the shifted linear vertical scale (322) of blood glucose levels.

2. The method of claim 1, wherein the display (300) includes a first color above the single smoothed curve and a different second color below the single smoothed curve;
and optionally
changing the first color, the second color, or both based on the current blood glucose level (330).

3. The method of one of claim 1, wherein the smoothed curve provides a non-numerical representation of the predicted future blood glucose level (350).

4. The method of claim 1, predicting a future blood glucose (350) level using a predictive algorithm that uses insulin delivery and/or food consumption data;
and optionally
wherein the predictive algorithm assumes a future basal insulin delivery that is adjusted based on a closed-loop insulin delivery algorithm.

5. The method of claim 1, wherein the smoothed curve and/or the point indicator provide an approximation of the predicted future blood glucose level (350).

6. The method of claim 1, further comprising:
presenting a blood glucose level representation that corresponds to the non-linear vertical scale (310), and
wherein the presented predicted future blood glucose (350) level is presented relative to the blood glucose level representation;
and optionally
wherein the blood glucose level representation comprises one or more of numerical representations of blood glucose levels and non-numerical representations of blood glucose levels;
and further optionally
wherein the blood glucose level representation comprises at least one region defined by a first numerical representation of a blood glucose level and a second numerical representation of a blood glucose level, and at least part of the presented predicted future blood glucose level (350) corresponds the at least one region.

7. The method of claim 1, wherein the non-linear vertical scale (310) of blood glucose values is a risk-based scale such that areas of greater risk receive more of the total space of the display (300).

8. A device (60) for displaying blood glucose information, comprising: a display (300);
one or more processors; and
a non-transitory computer-readable medium containing instructions that, when executed by the one or more processors, cause the device (60) to perform operations, the operations comprising:
monitoring blood glucose levels of a user, the blood glucose levels including at least a current blood glucose level (330) and a historic blood glucose level (340);
determining, by the one or more processors, a first location along a vertical axis of the display (300) for the current blood glucose level (330) by applying a non-linear vertical scale (310) to the current blood glucose level (330), the non-linear vertical scale (310) being a logarithmic scale such that lower blood glucose values are assigned to locations receiving logarithmically more space along the vertical axis of the display (300);
shifting, by the one or more processors, a linear vertical scale (322) of blood glucose levels along the vertical axis of the display (300) based on the first location such that the current blood glucose level (330) corresponds to the first location on the linear vertical scale (322);
determining, by the one or more processors, second locations along the vertical axis of the display (300) for the historic blood glucose level (340) and a predicted future blood glucose (350) level based on the shifted linear vertical scale (322) relative to the first location;
presenting the current blood glucose level (330) on the display (300) at the first location using a point indicator centered on a horizontal axis, wherein the point indicator is displayed according to the non-linear logarithmic scale; and
presenting the historic blood glucose level (340) and a predicted future blood glucose (350) level on the display (300) at the second locations as a single smoothed curve passing through the point indicator, wherein the historic blood glucose level (340) and the predicted future blood glucose (350) are displayed according to the shifted linear vertical scale (322) of blood glucose levels.

9. The device (60) of claim 8, wherein the display (300) includes a first color above the single smoothed curve and a different second color below the single smoothed curve;
and optionally
wherein the operations further comprise changing the first color, the second color, or both based on the current blood glucose level (330).

10. The device (60) of claim 8, wherein the smoothed curve is configured to provide a non-numerical representation of the predicted future blood glucose (350) level.

11. The device (60) of claims 8, wherein the operations further comprise predicting a future blood glucose (350) level using a predictive algorithm that uses insulin delivery and/or food consumption data;
and optionally
wherein the predictive algorithm assumes a future basal insulin delivery that is adjusted based on a closed-loop insulin delivery algorithm.

12. The device (60) of claim 8, wherein the smoothed curve and/or the point indicator provide an approximation of the predicted future blood glucose (350) level.

13. The device (60) of claim 8, wherein the operations further comprise:
presenting a blood glucose level representation that corresponds to the non-linear vertical scale (310) of blood glucose values, and
wherein the presented predicted future blood glucose (350) level is presented relative to the blood glucose level representation;
and optionally
wherein the blood glucose level representation comprises one or more of numerical representations of blood glucose levels and non-numerical representations of blood glucose levels;
and further optionally
wherein the blood glucose level representation comprises at least one region defined by a first numerical representation of a blood glucose level and a second numerical representation of a blood glucose level, and at least part of the presented predicted future blood glucose (350) level corresponds the at least one region.

14. The device (60) of claim 8, wherein the non-linear vertical scale (310) of blood glucose values is a risk-based scale such that areas of greater risk receive more of the total space of the display (300).

## Patentansprüche

1. Verfahren zum Anzeigen von Blutzuckerinformationen, wobei das Verfahren dies umfasst:
Überwachen von Blutzuckerspiegeln eines Benutzers, wobei die Blutzuckerspiegel wenigstens einen aktuellen Blutzuckerspiegel (330) und einen historischen Blutzuckerspiegel (340) aufweisen;
Bestimmen eines ersten Ortes entlang einer vertikalen Achse einer Anzeige (300) für den aktuellen Blutzuckerspiegel (330) durch Anwenden einer nicht-linearen vertikalen Skala (310) auf den aktuellen Blutzuckerspiegel (330), wobei die nicht-lineare vertikale Skala (310) eine logarithmische Skala ist, sodass niedrigere Blutzuckerwerte Orten zugewiesen werden, die logarithmisch mehr Raum entlang der vertikalen Achse der Anzeige (300) erhalten;
Verschieben einer linearen vertikalen Skala (322) von Blutzuckerspiegeln entlang der vertikalen Achse der Anzeige (300) basierend auf dem ersten Ort, sodass der aktuelle Blutzuckerspiegel (330) dem ersten Ort auf der linearen vertikalen Skala (322) entspricht;
Bestimmen zweiter Orte entlang der vertikalen Achse der Anzeige (300) für den historischen Blutzuckerspiegel (340) und einen vorhergesagten zukünftigen Blutzuckerspiegel (350) basierend auf der verschobenen linearen vertikalen Skala (322) relativ zu dem ersten Ort;
Präsentieren des aktuellen Blutzuckerspiegels (330) auf der Anzeige (300) an dem ersten Ort unter Verwendung eines Punktindikators, der auf einer horizontalen Achse zentriert ist, wobei der Punktindikator gemäß der nicht-linearen logarithmischen Skala angezeigt wird; und
Präsentieren des historischen Blutzuckerspiegels (340) und eines vorhergesagten zukünftigen Blutzuckerspiegels (350) auf der Anzeige (300) an den zweiten Orten als eine einzelne geglättete Kurve, die durch den Punktindikator verläuft, wobei der historische Blutzuckerspiegel (340) und der vorhergesagte zukünftige Blutzuckerspiegel (350) gemäß der verschobenen linearen vertikalen Skala (322) von Blutzuckerspiegeln angezeigt werden.

2. Verfahren nach Anspruch 1, wobei die Anzeige (300) eine erste Farbe über der einzelnen geglätteten Kurve und eine andere zweite Farbe unter der einzelnen geglätteten Kurve aufweist;
und optional
Ändern der ersten Farbe, der zweiten Farbe oder beider Farben basierend auf dem aktuellen Blutzuckerspiegel (330).

3. Verfahren nach Anspruch 1, wobei die geglättete Kurve eine nicht-numerische Darstellung des vorhergesagten zukünftigen Blutzuckerspiegels (350) bereitstellt.

4. Verfahren nach Anspruch 1, welches einen zukünftigen Blutzuckerspiegel (350) unter Verwendung eines Vorhersagealgorithmus vorhersagt, der Insulinabgabe- und/oder Nahrungsmittelverzehrdaten verwendet;
und optional
wobei der Vorhersagealgorithmus von einer zukünftigen Basalinsulinabgabe ausgeht, die basierend auf einem Insulinabgabealgorithmus mit geschlossenem Regelkreis angepasst wird.

5. Verfahren nach Anspruch 1, wobei die geglättete Kurve und/oder der Punktindikator eine Annäherung des vorhergesagten zukünftigen Blutzuckerspiegels (350) bereitstellen.

6. Verfahren nach Anspruch 1, ferner umfassend:
Präsentieren einer Blutzuckerspiegeldarstellung, die der nicht-linearen vertikalen Skala (310) entspricht, und
wobei der präsentierte vorhergesagte zukünftige Blutzuckerspiegel (350) relativ zu der Blutzuckerspiegeldarstellung präsentiert wird;
und optional
wobei die Blutzuckerspiegeldarstellung eines oder mehrere von numerischen Darstellungen von Blutzuckerspiegeln und nicht-numerischen Darstellungen von Blutzuckerspiegeln umfasst;
und ferner optional
wobei die Blutzuckerspiegeldarstellung wenigstens eine Region umfasst, die durch eine erste numerische Darstellung eines Blutzuckerspiegels und eine zweite numerische Darstellung eines Blutzuckerspiegels definiert ist, und wenigstens ein Teil des präsentierten vorhergesagten zukünftigen Blutzuckerspiegels (350) der wenigstens einen Region entspricht.

7. Verfahren nach Anspruch 1, wobei die nicht-lineare vertikale Skala (310) von Blutzuckerwerten eine risikobasierte Skala ist, sodass Bereiche mit einem größeren Risiko mehr von dem gesamten Raum der Anzeige (300) erhalten.

8. Vorrichtung (60) zum Anzeigen von Blutzuckerinformationen, umfassend:
eine Anzeige (300);
einen oder mehrere Prozessoren; und
ein nicht-transitorisches computerlesbares Medium, das Anweisungen enthält, die bei Ausführung durch die ein oder mehreren Prozessoren die Vorrichtung (60) dazu veranlassen, Operationen durchzuführen, wobei die Operationen dies umfassen:
Überwachen von Blutzuckerspiegeln eines Benutzers, wobei die Blutzuckerspiegel wenigstens einen aktuellen Blutzuckerspiegel (330) und einen historischen Blutzuckerspiegel (340) aufweisen;
Bestimmen, durch die ein oder mehreren Prozessoren, eines ersten Ortes entlang einer vertikalen Achse der Anzeige (300) für den aktuellen Blutzuckerspiegel (330) durch Anwenden einer nicht-linearen vertikalen Skala (310) auf den aktuellen Blutzuckerspiegel (330), wobei die nicht-lineare vertikale Skala (310) eine logarithmische Skala ist, sodass niedrigere Blutzuckerwerte Orten zugewiesen werden, die logarithmisch mehr Raum entlang der vertikalen Achse der Anzeige (300) erhalten;
Verschieben, durch die ein oder mehreren Prozessoren, einer linearen vertikalen Skala (322) von Blutzuckerspiegeln entlang der vertikalen Achse der Anzeige (300) basierend auf dem ersten Ort, sodass der aktuelle Blutzuckerspiegel (330) dem ersten Ort auf der linearen vertikalen Skala (322) entspricht;
Bestimmen, durch die ein oder mehreren Prozessoren, zweiter Orte entlang der vertikalen Achse der Anzeige (300) für den historischen Blutzuckerspiegel (340) und einen vorhergesagten zukünftigen Blutzuckerspiegel (350) basierend auf der verschobenen linearen vertikalen Skala (322) relativ zu dem ersten Ort;
Präsentieren des aktuellen Blutzuckerspiegels (330) auf der Anzeige (300) an dem ersten Ort unter Verwendung eines Punktindikators, der auf einer horizontalen Achse zentriert ist, wobei der Punktindikator gemäß der nicht-linearen logarithmischen Skala angezeigt wird; und
Präsentieren des historischen Blutzuckerspiegels (340) und eines vorhergesagten zukünftigen Blutzuckerspiegels (350) auf der Anzeige (300) an den zweiten Orten als eine einzelne geglättete Kurve, die durch den Punktindikator verläuft, wobei der historische Blutzuckerspiegel (340) und der vorhergesagte zukünftige Blutzuckerspiegel (350) gemäß der verschobenen linearen vertikalen Skala (322) von Blutzuckerspiegeln angezeigt werden.

9. Vorrichtung (60) nach Anspruch 8, wobei die Anzeige (300) eine erste Farbe über der einzelnen geglätteten Kurve und eine andere zweite Farbe unter der einzelnen geglätteten Kurve aufweist;
und optional
wobei die Operationen ferner das Ändern der ersten Farbe, der zweiten Farbe oder beider Farben basierend auf dem aktuellen Blutzuckerspiegel (330) umfasst.

10. Vorrichtung (60) nach Anspruch 8, wobei die geglättete Kurve dazu ausgelegt ist, eine nicht-numerische Darstellung des vorhergesagten zukünftigen Blutzuckerspiegels (350) bereitzustellen.

11. Vorrichtung (60) nach Anspruch 8, wobei die Operationen ferner das Vorhersagen eines zukünftigen Blutzuckerspiegels (350) unter Verwendung eines Vorhersagealgorithmus umfassen, der Insulinabgabe- und/oder Nahrungsmittelverzehrdaten verwendet;
und optional
wobei der Vorhersagealgorithmus von einer zukünftigen Basalinsulinabgabe ausgeht, die basierend auf einem Insulinabgabealgorithmus mit geschlossenem Regelkreis angepasst wird.

12. Vorrichtung (60) nach Anspruch 8, wobei die geglättete Kurve und/oder der Punktindikator eine Annäherung des vorhergesagten zukünftigen Blutzuckerspiegels (350) bereitstellen.

13. Vorrichtung (60) nach Anspruch 8, wobei die Operationen ferner dies umfassen:
Präsentieren einer Blutzuckerspiegeldarstellung, die der nicht-linearen vertikalen Skala (310) von Blutzuckerwerten entspricht, und
wobei der präsentierte vorhergesagte zukünftige Blutzuckerspiegel (350) relativ zu der Blutzuckerspiegeldarstellung präsentiert wird;
und optional
wobei die Blutzuckerspiegeldarstellung eines oder mehrere von numerischen Darstellungen von Blutzuckerspiegeln und nicht-numerischen Darstellungen von Blutzuckerspiegeln umfasst;
und ferner optional
wobei die Blutzuckerspiegeldarstellung wenigstens eine Region umfasst, die durch eine erste numerische Darstellung eines Blutzuckerspiegels und eine zweite numerische Darstellung eines Blutzuckerspiegels definiert ist, und wenigstens ein Teil des präsentierten vorhergesagten zukünftigen Blutzuckerspiegels (350) der wenigstens eine Region entspricht.

14. Vorrichtung (60) nach Anspruch 8, wobei die nicht-lineare vertikale Skala (310) von Blutzuckerwerten eine risikobasierte Skala ist, sodass Bereiche mit einem größeren Risiko mehr von dem gesamten Raum der Anzeige (300) erhalten.

## Revendications

1. Procédé d'affichage d'informations sur la glycémie, le procédé comprenant :
la surveillance des niveaux de glycémie d'un utilisateur, les niveaux de glycémie comprenant au moins un niveau de glycémie actuel (330) et un niveau de glycémie historique (340) ;
la détermination d'un premier emplacement le long d'un axe vertical d'un afficheur (300) pour le niveau de glycémie actuel (330) en appliquant une échelle verticale non linéaire (310) au niveau de glycémie actuel (330), l'échelle verticale non linéaire (310) étant une échelle logarithmique de sorte que des valeurs de glycémie inférieures soient attribuées à des emplacements recevant logarithmiquement plus d'espace le long de l'axe vertical de l'afficheur (300) ;
le décalage d'une échelle verticale linéaire (322) des niveaux de glycémie le long de l'axe vertical de l'afficheur (300) sur la base du premier emplacement de sorte que le niveau de glycémie actuel (330) corresponde au premier emplacement sur l'échelle verticale linéaire (322) ;
la détermination de seconds emplacements le long de l'axe vertical de l'afficheur (300) pour le niveau de glycémie historique (340) et un niveau de glycémie futur prédit (350) sur la base de l'échelle verticale linéaire décalée (322) par rapport au premier emplacement ;
la présentation du niveau de glycémie actuel (330) sur l'afficheur (300) au premier emplacement à l'aide d'un indicateur ponctuel centré sur un axe horizontal, l'indicateur ponctuel étant affiché en fonction de l'échelle logarithmique non linéaire ; et
la présentation du niveau de glycémie historique (340) et d'un niveau de glycémie futur prédit (350) sur l'afficheur (300) aux seconds emplacements sous la forme d'une courbe lissée unique passant par l'indicateur ponctuel, le niveau de glycémie historique (340) et le niveau de glycémie futur prédit (350) étant affichés en fonction de l'échelle verticale linéaire décalée (322) des niveaux de glycémie.

2. Procédé selon la revendication 1, l'afficheur (300) comprenant une première couleur au-dessus de la courbe lissée unique et une seconde couleur différente au-dessous de la courbe lissée unique ;
et éventuellement
la modification de la première couleur, de la seconde couleur ou des deux sur la base du niveau de glycémie actuel (330).

3. Procédé selon la revendication 1, la courbe lissée fournissant une représentation non numérique du niveau de glycémie futur prédit (350).

4. Procédé selon la revendication 1, prédisant un niveau de glycémie futur (350) en utilisant un algorithme prédictif qui utilise des données d'administration d'insuline et/ou de consommation d'aliment ;
et éventuellement
l'algorithme prédictif supposant une administration future d'insuline basale qui est ajustée sur la base d'un algorithme d'administration d'insuline en boucle fermée.

5. Procédé selon la revendication 1, la courbe lissée et/ou l'indicateur ponctuel fournissant une approximation du niveau de glycémie futur prédit (350).

6. Procédé selon la revendication 1, comprenant en outre :
la présentation d'une représentation de niveau de glycémie correspondant à l'échelle verticale non linéaire (310), et
le niveau de glycémie futur prédit (350) présenté étant présenté par rapport à la représentation de niveau de glycémie ;
et éventuellement
la représentation de niveau de glycémie comprenant une ou plusieurs représentations numériques des niveaux de glycémie et des représentations non numériques des niveaux de glycémie ;
et en outre éventuellement
la représentation de niveau de glycémie comprenant au moins une région définie par une première représentation numérique d'un niveau de glycémie et une seconde représentation numérique d'un niveau de glycémie, et au moins une partie du niveau de glycémie futur prédit présenté (350) correspondant à l'au moins une région.

7. Procédé selon la revendication 1, l'échelle verticale non linéaire (310) des valeurs de glycémie étant une échelle basée sur le risque telle que les zones à risque plus élevé reçoivent plus de l'espace total de l'afficheur (300).

8. Dispositif d'affichage (60) d'informations sur la glycémie, comprenant :
un afficheur (300) ;
un ou plusieurs processeurs ; et
un support lisible par ordinateur non transitoire contenant des instructions qui, lorsqu'elles sont exécutées par le ou les processeurs, amènent le dispositif (60) à effectuer des opérations, les opérations comprenant :
la surveillance des niveaux de glycémie d'un utilisateur, les niveaux de glycémie comprenant au moins un niveau de glycémie actuel (330) et un niveau de glycémie historique (340) ;
la détermination, par le ou les processeurs, d'un premier emplacement le long d'un axe vertical de l'afficheur (300) pour le niveau de glycémie actuel (330) en appliquant une échelle verticale non linéaire (310) au niveau de glycémie actuel (330), l'échelle verticale non linéaire (310) étant une échelle logarithmique de sorte que des valeurs de glycémie inférieures sont attribuées à des emplacements recevant logarithmiquement plus d'espace le long de l'axe vertical de l'afficheur (300) ;
le décalage, par le ou les processeurs, d'une échelle verticale linéaire (322) des niveaux de glycémie le long de l'axe vertical de l'afficheur (300) sur la base du premier emplacement de sorte que le niveau de glycémie actuel (330) corresponde au premier emplacement sur l'échelle verticale linéaire (322) ;
la détermination, par le ou les processeurs, de seconds emplacements le long de l'axe vertical de l'afficheur (300) pour le niveau de glycémie historique (340) et un niveau de glycémie futur prédit (350) sur la base de l'échelle verticale linéaire décalée (322) par rapport au premier emplacement ;
la présentation du niveau de glycémie actuel (330) sur l'afficheur (300) au premier emplacement à l'aide d'un indicateur ponctuel centré sur un axe horizontal, l'indicateur ponctuel étant affiché en fonction de l'échelle logarithmique non linéaire ; et
la présentation du niveau de glycémie historique (340) et d'un niveau de glycémie futur prédit (350) sur l'afficheur (300) aux seconds emplacements sous la forme d'une courbe lissée unique passant par l'indicateur ponctuel, le niveau de glycémie historique (340) et le niveau de glycémie futur prédit (350) étant affichés en fonction de l'échelle verticale linéaire décalée (322) des niveaux de glycémie.

9. Dispositif (60) selon la revendication 8, l'afficheur (300) comprenant une première couleur au-dessus de la courbe lissée unique et une seconde couleur différente au-dessous de la courbe lissée unique ;
et éventuellement
les opérations comprenant en outre la modification de la première couleur, de la seconde couleur ou des deux sur la base du niveau de glycémie actuel (330).

10. Dispositif (60) selon la revendication 8, la courbe lissée étant configurée pour fournir une représentation non numérique du niveau de glycémie futur prédit (350).

11. Dispositif (60) selon la revendication 8, les opérations comprenant en outre la prédiction d'un niveau de glycémie futur (350) à l'aide d'un algorithme prédictif qui utilise des données d'administration d'insuline et/ou de consommation d'aliment ;
et éventuellement
l'algorithme prédictif supposant une administration future d'insuline basale qui est ajustée sur la base d'un algorithme d'administration d'insuline en boucle fermée.

12. Dispositif (60) selon la revendication 8, la courbe lissée et/ou l'indicateur ponctuel fournissant une approximation du niveau de glycémie futur prédit (350).

13. Dispositif (60) selon la revendication 8, les opérations comprenant en outre :
la présentation d'une représentation de niveau de glycémie correspondant à l'échelle verticale non linéaire (310) des valeurs de glycémie, et
le niveau de glycémie futur prédit (350) présenté étant présenté par rapport à la représentation de niveau de glycémie ;
et éventuellement
la représentation de niveau de glycémie comprenant une ou plusieurs parmi des représentations numériques des niveaux de glycémie et des représentations non numériques des niveaux de glycémie ;
et en outre éventuellement
la représentation de niveau de glycémie comprenant au moins une région définie par une première représentation numérique d'un niveau de glycémie et une seconde représentation numérique d'un niveau de glycémie, et au moins une partie du niveau de glycémie futur prédit présenté (350) correspondant à l'au moins une région.

14. Dispositif (60) selon la revendication 8, l'échelle verticale non linéaire (310) des valeurs de glycémie étant une échelle basée sur le risque telle que les zones à risque plus élevé reçoivent plus de l'espace total de l'afficheur (300).
